# EUROPEAN PATENT APPLICATION

(11) **EP 1 731 190 A1**
(43) Date of publication of application: **13.12.2006**
(21) Application number: 06010159.9
(22) Date of filing: 17.05.2006
(51) Int. Cl.: A61M 25/00

(54) **Assembly of a vascular drive device and a removable stiffening means**

(30) Priority: 31.05.2005 IT MI20051012
(71) Applicant: BIOENGINEERING LABORATORIES S.p.A., 22063 Cantu' CO (IT)
(72) Inventor: Galli, Franco, . (IT); Greco, Francesco, . (IT)
(74) Representative: Ferraiolo, Ruggero

(57) **Abstract**

The outer wall of a stiffenig cannula (2) is a discontinuous wall defined by the top of a plurality of projections (4b) extending from the cannula base wall in order to reduce the overall contact surface between the catheter (1) and the cannula (2) so making easy the drawing of the cannula from the catheter once the latter is in the operation location (Fig3).

## Description

The invention lies in the field of the vascular drive devices, particularly in the field of the central venous catheters.

Seldingher procedure for introducing a catheter into a vein hollow is still the most appropriate one. This procedure comprises the means and steps specified in the following : a) introducing a hollow needle through the skin of a patient until the distal end of the needle enters the hollow of the selected vein; b) introducing a guide wire into needle hole until the distal end of the wire is in the vein hollow; c) drawing out the needle completely; d) introducing and then drawing out a dilator which opens up the hole produced in the skin and in the vein wall by the needle; e) guiding a catheter along the guide wire until the catheter is in the vein hollow through the holes in the skin and in the vein; f) drawing out the guide wire.

The procedure which is described hereabove is good provided the catheter used is not too flexible and soft in which case the use of a stiffening cannula introduced into the catheter hollow is required in order to guide the catheter in the skin hole and then in the vein hole. An assembly catheter-cannula is so generated which has such a stiffness as to allow the introduction of the catheter without problems.

The drawback of the described procedure with small catheters diameters is that the drawing out of the the stiffening cannula from the catheter proves to be difficult and sometimes prevented because of the close tolerance between the catheter and the cannula. The latter remains so tight within the catheter inner wall that the friction between said two pieces may be won thanks only to efforts and movements which result detrimental for the operation.

The invented assembly obviates the above drawback. The assembly comprises one catheter and a hollow stiffening cannula and is characterized in that the outer surface of the stiffening cannula is a discontinuous surface defined by the top of a plurality of projections extending from the base wall to reduce the overall contact between the catheter and cannula. Said feature makes easy to remove the cannula from the catheter once the latter is placed in its working location with the distal end into the vein hollow so that, significantly, the operator may proceed with his operation. The piece that last may be drawn out from the catheter is the cannula or the guide wire.

The advantage of this assembly comprising the catheter and the cannula is in that it permits to use exceptionally flexible and soft catheters and also to introduce the catheter without using a dilator.

The invention is described in detail by an example of a preferred embodiment and the attached drawings where the parts appear much enlarged with respect to the real dimensions in order to make clear the figures in which:
- Fig. 1 is a perspective view partly sectioned,
- Fig. 2 is a part perspective view,
- Fig. 3 is a partly sectioned part view,
- Fig. 4 is a first cross section and
- Fig. 5 is a second cross section.

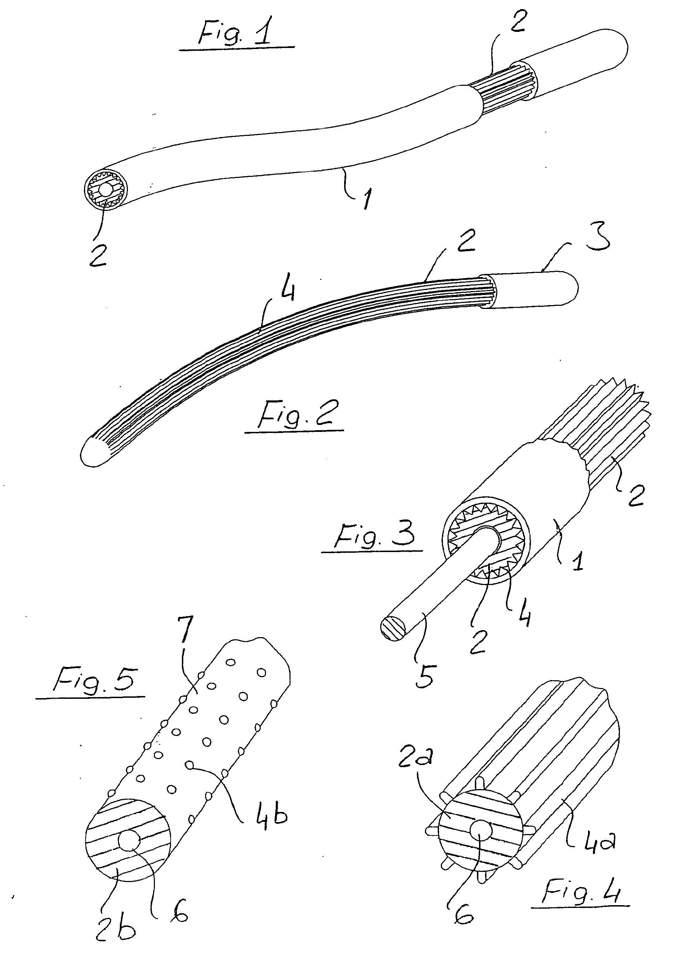

Fig. 1 shows a catheter 1 made of polyurethane having the outer diameter of 3.3 mm, the inner diameter of 2.2 mm and the length of 500 mm. It is very flexible and therefore it is provided with a stiffening cannula 2 that, as hown at the left sectioned end, bears a plurality of longitudinal riflings on the base wall, well shown as 4 in Fig. 3, which riflings have triangular cross section, each with the outer upper end lying on a diameter substantially coincident with the inner diameter of the catheter, the cannula being also provided with a central longitudinal hole adapted to receive a metal guide wire , shown as 5 in Fig. 3.

Fig. 2 shows the stiffening cannula 2 having rounded the distal end in order to make easy the introduction into the catheter and having enlarged the proximal end to define a sort of handle 3 for the operator. The plurality of longitudinal riflings is roughly indicated by lines 4.

Fig. 3 shows a part of the catheter 1 and of the stiffening cannula 2 with its longitudinal riflings of triangular section where each triangle has a base on the cannula base wall and a vertex in contact with the catheter inner wall and shows a part of the guide steel wire 5 passing through the cannula central hole 6 (see Fig. 4).

Fig. 4 shows a cannula 2a having a plurality of longitudinal riflings defined by extensions 4a of rectangular section with rounded top, perimetrically spaced each other and having the outer ends on a diameter substantially equal with the catheter inner diameter.

Fig. 5 shows a cannula 2b with its central hole 6 and a plurality of hemispherical extensions 4b made on the surface defined by the cannula base wall 7.

It is realized that the plurality of riflings may be made of extensions having different cross sections, adjacent one with other or spaced one from other, and may have a progression not strictly longitudinal, but for instance helicoidal, provided are granted both the reduction of the contact surface between the catheter inner wall and the stiffening cannula outer wall as well as a sufficient stiffness of the assembly.

## Claims

1. Assembly of a vascular drive device (1) and a removable stiffening means (2) defined by a catheter (1) and a stiffening hollow cannula (2) receivable in the catheter in order to stiffen the assembly when the operation is in course **characterized in that** the outer wall of the stiffening cannula (2) is a discontinuos wall defined by the top of a plurality of projections (4), each having the base on the cannula base wall (7) and the top on a diameter substantially coincident with the catheter inner diameter.

2. Assembly of a vascular drive device (1) and a removable stiffening means (2) according to claim 1 **characterized in that** the discontinuous wall is defined by the top of longitudinal riflings (4) of triangular cross section, parallel and adjacent one another.

3. Assembly of a vascular drive device (1) and a removable stiffening means (2) according to claim 2 **characterized in that** the longitudinal riflings are spaced one from another.

4. Assembly of a vascular drive device (1) and a removable stiffening means (2) according to claim 1 **characterized in that** the discontinuous wall is defined by the top of a plurality of longitudinal riflings of square or rectangular cross section.

5. Assembly of a vascular drive device (1) and a removable stiffening means (2) according to claim 4 **characterized in that** the riflings have rounded upper ends (4a).

6. Assembly of a vascular drive device (The outer wall of the stiffening 1) and a removable stiffening means (2) according to claim 1 **characterized in that** the discontinuous wall is defined by the top of a plurality of cylindrical projections with rounded top.

7. Assembly of a vascular drive device (1) and a removable stiffening means (2) according to claim 1 **characterized in that** the discontinuous wall is defined by the top of a plurality of hemispheric projections (4b) .

## Amended claims

### Amended claims in accordance with Rule 86(2) EPC.

**1.** Assembly of a vascular drive device (1) and a removable stiffening means (2) defined by a catheter (1) and a stiffening hollow cannula (2) receivable in the catheter in order to stiffen the assembly and reduce the friction between the inner catheter surface and the outer cannula surface**characterized in that** the outer wall of the stiffening cannula (2) is a discontinuos wall defined by the top of a plurality of substantially longitudinal projections (4, 4a), each having the base on the cannula base wall (7) and the top on a diameter substantially coincident with the catheter inner diameter.

**2.** Assembly of a vascular drive device (1) and a removable stiffening means (2) according to claim 1 **characterized in that** said projections are riflings (4) of triangular cross section, parallel and adjacent one another.

**3.** Assembly of a vascular drive device (1) and a removable stiffening means (2) according to claim 2 **characterized in that** the riflings (4a) are spaced one from another.

**4.** Assembly of a vascular drive device (1) and a removable stiffening means (2) according to claim 1 **characterized in that** the projections (4a) are of square or rectangular cross section.

**5.** Assembly of a vascular drive device (1) and a removable stiffening means (2) according to claim 4 **characterized in that** the projections (4a) have rounded upper ends.
